# FASCICULE DE BREVET EUROPEEN

(11) **EP 1 075 515 B1**
(45) Date de publication et mention de la délivrance du brevet: **31.08.2005**
(21) Numéro de dépôt: 98924390.2
(22) Date de dépôt: 06.05.1998
(51) Int. Cl.: C12N 15/10

(54) **PROCEDE DE CONSERVATION DE LONGUE DUREE DE MOLECULES D'ADN ET CONDITIONNEMENT POUR SA MISE EN OEUVRE**
VERFAHREN ZUR LANGZEITLAGERUNG VON DNS-MOLEKÜLEN, UND VERPACKUNG ZUR DURCHFÜHRUNG DIESES VERFAHRENS
METHOD FOR PROLONGED STORAGE OF DNA MOLECULES AND PACKAGING IMPLEMENTING SAID METHOD

(43) Date de publication de la demande: 14.02.2001
(73) Titulaire: Société IMAGENE, 33600 Pessac (FR)
(72) Inventeur: Tuffet, Sophie, 33800 Bordeaux (FR); De Souza, David Georges, 33800 Bordeaux (FR); Bonnet, Jacques, 33600 Pessac (FR); Campet, Guy, 33610 Canejan (FR); Noel, Thierry, 69003 Lyon (FR)
(74) Mandataire: Thébault, Jean-Louis
(86) Numéro de dépôt international: PCT/FR1998/000912
(87) Numéro de publication internationale: WO 1999/057264

(56) Documents cités:
- EP-A- 0 166 529
- EP-A- 0 242 294
- EP-A- 0 383 569
- WO-A-90/03036
- WO-A-90/03959
- DE-A- 4 119 574
- DE-U- 29 715 707
- FR-A- 2 755 440
- US-A- 3 200 085
- US-A- 3 609 372
- US-A- 4 806 368
- US-A- 5 565 318

## Description

La présente invention a trait à l'ADN (Acide désoxyribonucléique) notamment d'origine humaine, et vise à conserver la molécule d'ADN, laquelle est porteuse des gènes caractéristiques de chaque individu, c'est-à-dire de son patrimoine génétique.

Plus précisément, l'invention vise la sauvegarde de l'information génétique par la conservation de la molécule d'ADN de façon aussi prolongée que possible et dans des conditions de préservation de l'intégrité de l'information génétique. Cette invention présente de nombreux intérêts notamment pour la médecine prédictive, pour la généalogie génétique et l'identification.

Si la molécule d'ADN est relativement stable, des études d'archéogénéticiens ayant révélé qu'elle peut se conserver des millions d'années dans un environnement favorable, elle peut cependant être dégradée en l'absence de conservation dans un tel environnement.

Parmi les causes d'altération de l'ADN, on peut citer l'action des radiations ionisantes telles que les rayons X ou gamma, l'action des rayonnements ultraviolets, l'oxydation et l'hydrolyse enzymatique ou chimique.

La présente invention vise à proposer une technique de conservation de l'ADN dans un environnement le préservant des effets des actions ci-dessus.

A cet effet, l'invention a pour objet un procédé de conservation de longue durée de molécules d'ADN qui, dans un premier mode de mise en oeuvre, consiste, après extraction et purification de l'ADN par toute technique appropriée, conventionnelle ou non, à effectuer une encapsulation dans une atmosphère constituée d'un ou plusieurs gaz inertes et présentant un degré d'humidité inférieur ou égal à 1 ppm d'eau, de la molécule d'ADN préalablement déshumidifiée dans une capsule métallique, inoxydable, étanche.
Suivant un deuxième mode de mise en oeuvre, le procédé consiste, après extraction et purification de l'ADN par toute technique appropriée, conventionnelle ou non, à effectuer une encapsulation dans une capsule métallique, inoxydable, étanche, de la molécule d'ADN préalablement déshumidifiée, l'ADN étant, avant ladite encapsulation physique, soumis à une encapsulation chimique par enrobage dans un (co)polymère approprié.

Suivant une variante de ce deuxième mode de mise en oeuvre, l'encapsulation chimique est effectuée à l'aide d'un hybride constitué dudit (co)polymère, de molécules organiques et/ou de sels inorganiques, à des fins de protection renforcée de l'ADN à l'égard des rayonnements ultraviolets ou ionisants.

De préférence, et quel que soit le mode de mise en oeuvre du procédé, l'encapsulage physique est complétée par la mise de ladite capsule métallique inoxydable et étanche dans un conteneur résistant aux chocs et à l'écrasement.

L'ADN ainsi encapsulé peut être conservé théoriquement pendant plusieurs dizaines de milliers d'années, à l'abri des radiations ionisantes, des ultraviolets, des agressions chimiques et des contraintes mécaniques.

L'invention a également pour objet les différents types de conditionnement obtenus conformément au procédé, lesquels se présentent sous la forme soit d'une capsule seule, soit d'une capsule enfermée dans une enveloppe protectrice appelée conteneur.

A l'intérieur de la capsule est conservé une quantité d'ADN, par exemple 30 µg, suffisante pour effectuer un nombre substantiel de prises d'échantillons à tout moment.

L'ADN est déposé directement sur la capsule, ou sur une coupelle de verre introduite dans la capsule.

A chaque prise d'échantillon, la capsule est ouverte, la quantité désirée d'ADN est prélevée, le reste étant laissé dans la capsule, le conditionnement étanche étant reconstitué. L'ADN prélevé est ensuite réhydraté à des fins d'analyse.

Un tel conditionnement est de nature à assurer une préservation pérenne du patrimoine génétique qui se trouve à l'abri notamment de l'oxydation et des rayonnements ionisants et ultraviolets ainsi que d'autres agressions chimiques ou mécaniques.

D'autres caractéristiques et avantages ressortiront de la description qui va suivre de modes de mise en oeuvre du procédé de l'invention, description donnée à titre d'exemple uniquement et en regard du dessin annexé sur lequel la figure unique illustre schématiquement une structure d'un conditionnement conforme à l'invention.

Préalablement à la mise en oeuvre de l'invention, l'ADN est extrait et purifié.

Ceci peut être opéré par toute méthode conventionnelle ou non. L'ADN peut être extrait à partir de n'importe quelles cellules de l'organisme.

A titre d'exemple, l'ADN est extrait à partir du sang ou de bulbes de cheveux, de cellules issues de la salive, de muqueuses ou de cellules de la peau. On met en oeuvre ensuite un processus de purification à plusieurs étapes, à savoir : désagrégation des cellules, élimination des protéines par digestion enzymatique, isolement/extraction de l'ADN, amplification par polymérisation, si nécessaire, et conservation à l'abri des facteurs d'altération.

L'ADN ainsi préparé se présente sous la forme d'un précipité dans de l'alcool.

Le protocole ci-dessus est bien connu et peut être remplacé par tout autre procédé, existant ou nouveau.

L'ADN est alors, conformément à l'invention, placé dans une capsule métallique, inoxydable et étanche. Une telle encapsulation dite physique est effectuée dans une atmosphère constituée d'un ou plusieurs gaz inertes, tels que des gaz rares et présentant un degré d'humidité très faible, de préférence inférieur à 1 ppm d'eau, ladite atmosphère étant à la pression atmosphérique.

Ceci est opéré par exemple à l'aide d'une boîte sèche conventionnelle.

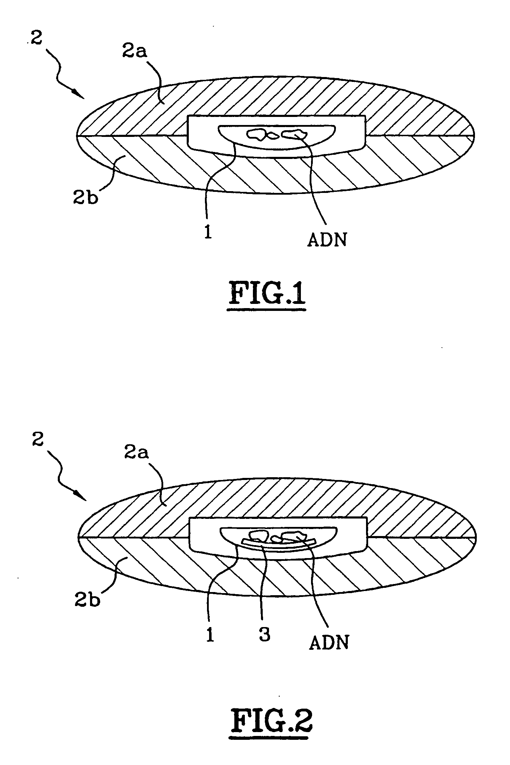

La capsule, représentée schématiquement en 1 sur la figure 1 du dessin annexé, est par exemple une capsule d'or formée d'une petite cuvette circulaire fermée par un opercule serti à la presse à la périphérie de la cuvette.

Le diamètre de la capsule d'or est par exemple de l'ordre de 5 mm et son épaisseur de 2 à 3 mm.

La solidarisation étanche de la cuvette et de son opercule peut être effectuée par tout autre moyen approprié.

L'or est préférable à cause de sa malléabilité et de ses propriétés d'inoxydabilité et de non-contamination de l'ADN. En variante, un alliage à base d'or ou de platine peut être utilisé.

L'atmosphère lors de l'encapsulation est desséchée afin d'éviter l'hydrolyse et l'oxydation de l'ADN après sertissage.

La quantité d'ADN placée dans la capsule, par exemple une trentaine de microgrammes, est largement suffisante pour autoriser un nombre substantiel de prélèvements distincts d'ADN dans la capsule au cours de sa conservation.

Un tel conditionnement peut suffire pour pouvoir assurer la préservation pérenne du patrimoine génétique pendant des dizaines, voire des centaines de milliers d'années, dans la mesure bien entendu où ladite capsule reste intègre.

Ce conditionnement protège l'ADN en particulier vis à vis :
- des réactions d'hydrolyses chimiques ou enzymatiques ;
- de coupures induites par des rayonnements ultraviolets ou toute radiation ionisante telle que rayons X ou gamma ;
- de l'oxydation par l'oxygène de l'air.

Avantageusement et pour renforcer la préservation de l'ADN, la capsule 1, à savoir ladite capsule d'or, est elle-même enfermée dans un conteneur étanche 2 en un matériau présentant de bonnes propriétés mécaniques, de façon à mieux protéger la capsule 1 vis à vis d'agressions mécaniques, en particulier les vibrations, les chocs et l'écrasement, ou toute autre agression, par exemple une élévation de température et, d'une manière générale, de façon à protéger ladite capsule contre l'environnement, qu'il soit normal ou anormal.

Le conteneur 2 peut être une sorte de petit boîtier en deux parties 2a, 2b, scellées ou solidarisées par tout autre moyen pour assurer l'intégrité et l'étanchéité de l'ensemble.

Le conteneur 2 peut être par exemple en un matériau approprié, par exemple céramique, composite, métallique, ou polymère.

Suivant un autre mode de mise en oeuvre du procédé de l'invention, l'ADN dûment préparé est, avant mise en place dans la capsule 1, soumis à une encapsulation dite chimique.

A cet effet, l'ADN est enrobé dans un polymère ou copolymère inerte vis à vis de l'ADN, à des fins de renforcement de la protection à l'égard des facteurs d'altération, les molécules d'ADN étant à l'abri dans les pores du (co)polymère.

Le matériau d'enrobage est choisi de telle sorte qu'il puisse être dissous ultérieurement afin de pouvoir récupérer les molécules d'ADN.

Tout (co)polymère peut convenir, excepté ceux risquant de réagir contre la molécule ADN, ceux qui empêcheront la redissolution ultérieure de l'ADN et ceux qui nécessitent pour une telle redissolution un solvant acide, c'est-à-dire ayant un pH inférieur ou égal à 4 environ.

On peut réaliser une telle encapsulation chimique en plaçant le matériau ADN dûment préparé, par exemple une pelote d'ADN obtenue par précipitation, dans une solution d'acide acrylique ou polyacrylique dans de l'alcool méthylique.

Par exemple, on met en solution dans 50 cm³ d'alcool méthylique 1 g d'acide acrylique ou polyacrylique. L'alcool est lentement évaporé jusqu'à l'obtention d'un gel visqueux. Un amas d'ADN d'environ 1 mm³ en suspension dans une solution alcoolique est placé au sein de ce gel.

L'ensemble est desséché à 50°C jusqu'à l'obtention d'un bloc solide contenant l'ADN, lequel est ensuite mis en place dans la capsule 1 suivant le processus exposé plus haut, avec cette différence qu'il n'est plus nécessaire d'opérer dans une boîte sèche, l'atmosphère neutre étant maintenue.

Ultérieurement et à tout moment, après ouverture des enveloppes 1 et 2, l'ADN peut être désencapsulé par immersion pendant 1 heure dans de l'alcool éthylique. On obtient à nouveau un amas de molécules identique à celui obtenu par le processus de purification.

Suivant un autre exemple mettant en oeuvre un autre (co)polymère, on prépare une solution de 1 g de métacrylate ou polymétacrylate de méthyle dissous dans 50 cm³ de dichlorométhane. Le solvant est évaporé jusqu'à obtention d'un gel visqueux. Un amas d'ADN d'environ 1 mm³ en suspension dans une solution alcoolique est placé au sein de ce gel. L'ensemble est desséché à 50°C jusqu'à obtention d'un bloc solide contenant l'ADN, lequel est ensuite mis en place dans la capsule 1 dans les mêmes conditions que l'exemple précédent.

Ultérieurement et à tout moment, après ouverture des enveloppes 1 et 2, l'ADN initial peut être régénéré par dissolution du (co)polymère par le dichlorométhane.

Suivant une variante de telles encapsulations chimiques, le (co)polymère est associé à des molécules organiques et/ou des sels inorganiques à des fins de meilleure protection vis à vis des rayonnements ultraviolets et des radiations ionisantes. On peut notamment ajouter au (co)polymère des ions de métaux lourds dans la mesure toutefois où il n'y a pas de risques de lésion de l'ADN.

Suivant un exemple, on dissous 1 g d'acrylamide ou de polyacrylamide dans 25 cm³ d'eau distillée. On ajoute à la solution 50 mg d'acétate de cuivre et 50 mg d'acétate de zinc. L'eau est évaporée lentement jusqu'à obtention d'un gel visqueux. Un amas d'ADN d'environ 1 mm³ en suspension dans une solution alcoolique est placé au sein de ce gel. L'ensemble est desséché à 50°C jusqu'à obtention d'un bloc solide contenant l'ADN, lequel est ensuite mis en place dans la capsule 1 dans les mêmes conditions que les exemples précédents.

Il est à noter que l'encapsulation chimique à l'aide d'un (co)polymère présente l'avantage de réaliser une polymérisation en billes individuelles, ce qui rend plus pratique les prélèvements ultérieurs d'ADN, puisqu'il suffit de retirer de la capsule une bille, sans toucher aux autres.

Comme polymère utilisable selon l'invention, on peut également citer l'agarose.

Enfin, l'invention n'est évidemment pas limitée aux modes de mise en oeuvre illustrés ci-dessus mais en couvre au contraire toutes les variantes, notamment en ce qui concerne la nature des matériaux constitutifs des capsules 1 ou conteneurs 2, les conditions d'encapsulations physique et chimique, la nature du (co)polymère d'encapsulation chimique et de ses éventuels additifs, ainsi que les forme et dimensions desdites capsules 1 ou conteneurs 2.

C'est ainsi qu'en variante l'ADN peut être déposé sur une coupelle de verre, notamment un verre sodo-calcique, telle que celle schématisée en 3 sur la figure 2 du dessin annexé.

L'ADN adhère au verre de la coupelle 3 qui peut présenter, à titre d'exemple, un diamètre de 7 mm et une hauteur de 1,2 mm, ladite coupelle 3 étant enfermée de manière étanche dans la capsule 1.

## Revendications

1. Procédé de conservation de longue durée de molécules d'ADN, **caractérisé en ce qu'**il consiste, après extraction et purification de l'ADN par toute technique appropriée, conventionnelle ou non, à effectuer une encapsulation dans une atmosphère constituée d'un ou plusieurs gaz inertes et présentant un degré d'humidité inférieur ou égal à 1 ppm d'eau, de la molécule d'ADN préalablement déshumidifiée dans une capsule métallique, inoxydable, étanche.

2. Procédé de conservation de longue durée de molécules d'ADN, **caractérisé en ce qu'**il consiste, après extraction et purification de l'ADN par toute technique appropriée, conventionnelle ou non, à effectuer une encapsulation dans une capsule métallique, inoxydable, étanche, de la molécule d'ADN préalablement déshumidifiée, l'ADN étant, avant ladite encapsulation physique, soumis à une encapsulation chimique par enrobage dans un (co)polymère approprié.

3. Procédé suivant la revendication 2, **caractérisé en ce que** l'encapsulation chimique est effectuée à l'aide d'un hybride constitué dudit (co)polymère, de molécules organiques et/ou de sels inorganiques, à des fins de protection renforcée de l'ADN à l'égard des rayonnements ultraviolets ou ionisants.

4. Procédé suivant la revendication 3, **caractérisé en ce que** l'hybride est constitué d'un (co)polymère et d'ions de métaux lourds,

5. Procédé suivant l'une des revendications 2 à 4, **caractérisé en ce que** le (co)polymère et ses éventuels additifs organiques ou inorganiques sont dissous dans un solvant, évaporé ensuite jusqu'à obtention d'un gel visqueux, puis un amas déterminé d'ADN en suspension dans une solution alcoolique est placé au sein dudit gel et l'ensemble est desséché à une température appropriée jusqu'à l'obtention d'un bloc solide contenant l'ADN.

6. Procédé suivant la revendication 5, **caractérisé en ce que** l'encapsulation chimique est suivie d'une encapsulation dans une capsule métallique, inoxydable, étanche, dans une atmosphère constituée d'un ou plusieurs gaz inertes.

7. Procédé suivant l'une des revendication 1 à 6, **caractérisé en ce que** ladite capsule est placée dans un conteneur étanche résistant aux chocs et à l'écrasement.

8. Procédé suivant l'une des revendications 1 à 7, **caractérisé en ce qu'**avant son encapsulage dans la capsule métallique, l'ADN est déposé sur une coupelle de verre.

9. Conditionnement de conservation obtenu conformément au procédé de l'une quelconque des revendications 1 à 6, **caractérisé en ce qu'**il est constitué d'une capsule en métal ou alliage métallique malléable et présentant des propriétés d'inoxydabilité et de non-contamination de l'ADN, fermée de manière étanche par un opercule par tous moyens appropriés, notamment un sertissage.

10. Conditionnement selon la revendication 9, plus particulièrement destiné à la mise en oeuvre du procédé de la revendication 7, **caractérisé en ce qu'**il est enfermé dans un conteneur dans un matériau choisi dans le groupe comprenant les céramiques, les composites, les métaux, les polymères.

11. Conditionnement selon la revendication 9 ou 10, plus particulièrement destiné à la mise en oeuvre du procédé de la revendication 8, **caractérisé en ce qu'**il comporte, à l'intérieur de la capsule métallique, une coupelle en verre sur laquelle est déposé l'ADN.

12. Conditionnement selon la revendication 11, **caractérisé en ce que** la coupelle est constituée d'un verre sodo-calcique.

## Patentansprüche

1. Verfahren zur Langzeitkonservierung von DNA-Molekülen, **dadurch gekennzeichnet, dass** es darin besteht, nach Extraktion und Reinigung der DNA durch jede geeignete Technik, konventioneller Art oder nicht, eine Verkapselung des vorher entfeuchteten DNA-Moleküls in einer metallischen, rostfreien, dichten Kapsel in einer Atmosphäre durchzuführen, die von einem oder mehreren Inertgasen gebildet ist und einen Feuchtigkeitsgrad kleiner oder gleich 1 ppm Wasser aufweist.

2. Verfahren zur Langzeitkonservierung von DNA-Molekülen, **dadurch gekennzeichnet, dass** es darin besteht, nach Extraktion und Reinigung der DNA durch jede geeignete Technik, konventioneller Art oder nicht, eine Verkapselung des vorher entfeuchteten DNA-Moleküls in einer metallischen, rostfreien, dichten Kapsel durchzuführen, wobei die DNA vor der physischen Verkapselung einer chemischen Verkapselung durch Ummanteln mit einem geeigneten (Co)polymer unterzogen wird.

3. Verfahren nach Anspruch 2, **dadurch gekennzeichnet, dass** die chemische Verkapselung mit Hilfe eines Hybrids erfolgt, das von dem (Co)polymer, organischen und/oder anorganischen Molekülen gebildet ist, um die DNA verstärkt gegen ultraviolette oder ionisierende Strahlen zu schützen.

4. Verfahren nach Anspruch 3, **dadurch gekennzeichnet, dass** das Hybrid von einem (Co)polymer und Schwermetallionen gebildet ist.

5. Verfahren nach einem der Ansprüche 2 bis 4, **dadurch gekennzeichnet, dass** das (Co)polymer und seine möglichen organischen oder anorganischen Zusätze in einem Lösungsmittel gelöst sind, das dann bis zum Erhalt eines viskosen Gels verdampft wird, und dass dann eine bestimmte Menge an DNA in Suspension in einer alkoholischen Lösung innerhalb des Gels angeordnet und die Gesamtheit bei einer geeigneten Temperatur bis zum Erhalt eines festen, die DNA enthaltenden Blocks getrocknet wird.

6. Verfahren nach Anspruch 5, **dadurch gekennzeichnet, dass** auf die chemische Verkapselung eine Verkapselung in einer metallischen, rostfreien, dichten Kapsel in einer von einem oder mehreren Inertgasen gebildeten Atmosphäre folgt.

7. Verfahren nach einem der Ansprüche 1 bis 6, **dadurch gekennzeichnet, dass** die Kapsel in einem dichten, gegen Stöße und Zusammendrücken festen Behälter angeordnet wird.

8. Verfahren nach einem der Ansprüche 1 bis 7, **dadurch gekennzeichnet, dass** die DNA vor ihrer Verkapselung in der metallischen Kapsel auf eine Glaskuppel gelegt wird.

9. Konservierungsverpackung, die gemäß dem Verfahren nach einem der Ansprüche 1 bis 6 erhalten wird, **dadurch gekennzeichnet, dass** sie von einer Kapsel aus Metall oder einer geschmeidigen Metalllegierung gebildet ist, Eigenschaften des Nichtrostens und der Nichtkontaminierung der DNA aufweist und dicht von einem Deckel durch jedes geeignete Mittel, insbesondere eine Crimpverbindung, verschlossen ist.

10. Verpackung nach Anspruch 9, die insbesondere für den Einsatz des Verfahrens des Anspruchs 7 bestimmt ist, **dadurch gekennzeichnet, dass** sie in einem Behälter aus einem Material eingeschlossen ist, das in der Gruppe ausgewählt wird, die die Keramikstoffe, Verbundstoffe, Metalle, Polymere enthält.

11. Verpackung nach Anspruch 9 oder 10, die insbesondere für den Einsatz des Verfahrens des Anspruchs 9 bestimmt ist, **dadurch gekennzeichnet, dass** sie im Inneren der metallischen Kapsel eine Glaskuppel umfasst, auf die die DNA gelegt wird.

12. Verpackung nach Anspruch 11, **dadurch gekennzeichnet, dass** die Kuppel von einem Alkali-Kalk-Glas gebildet ist.

## Claims

1. Process for the long-term preservation of DNA molecules, **characterized in that** it consists, after extraction and purification of the DNA by any suitable technique, conventional or otherwise, in carrying out an encapsulation in an atmosphere constituted by one or several inert gases and having a degree of humidity less than or equal to 1 ppm of water, of the previously dehumidified DNA molecule in a sealed corrosion proof metallic capsule (1).

2. Process according to claim 1, **characterized in that** before said physical encapsulation, the DNA is subjected to a chemical encapsulation by cladding with a suitable (co)polymer.

3. Process according to claim 2, **characterized in that** the chemical encapsulation is carried out with a hybrid constituted of said (co)polymer, of organic and/or inorganic salt molecules, for increased protection of the DNA with respect to ultraviolet or ionizing radiation's.

4. Process according to claim 3, **characterized in that** the hybrid is constituted of a (co)polymer and ions of heavy metal.

5. Process according to one of claims 2 to 4, **characterized in that** the (co)polymer and its possible organic or inorganic additives, are dissolved in a solvent, thereafter evaporated to obtain a viscous gel, then a predetermined mass of DNA in suspension in an alcohol solution is placed within said gel and the assembly is dried at a suitable temperature to obtain a solid block containing the DNA.

6. Process according to claim 5, **characterized in that** the chemical encapsulation is followed by an encapsulation in a sealed corrosion proof metallic capsule (1), in an atmosphere constituted by one or several inert gases.

7. Process according to one of claims 1 to 6, **characterized in that** said capsule (1) is disposed in a sealed container (2) resistant to shock and crushing.

8. Process according to one of claims 1 to 7, **characterized in that** before its encapsulation in the metallic capsule (1), the DNA is disposed in a glass dish.

9. Preservative packing obtained according to the process of any one of claims 1 to 7, **characterized in that** it is constituted of a capsule (1) of malleable metal or metallic alloy and having corrosion-proof and noncontaminating properties as to the DNA, closed in a sealed manner by a lid by any suitable means, particularly seating.

10. Packaging according to claim 9, more particularly adapted to practice the process of claim 8, **characterized in that** it is enclosed in a container (2) in a material selected from the group consisting of ceramics, composites, metals and polymers.

11. Packaging according to claim 9 or 10, more particularly adapted to practice the process of claim 9, **characterized in that** it comprises, within the metallic capsule (1), a glass dish (3) on which the DNA is disposed.

12. Packaging according to claim 11, **characterized in that** the dish (3) is constituted of a sodium-calcium glass.
